# EUROPEAN PATENT APPLICATION

(11) **EP 2 500 052 A1**
(43) Date of publication of application: **19.09.2012**
(21) Application number: 11305303.7
(22) Date of filing: 18.03.2011
(51) Int. Cl.: A61M 5/31, A61M 5/315

(54) **Container for improved injection**

(71) Applicant: Becton Dickinson France, 38800 Le Pont-de-Claix (FR)
(72) Inventor: Jouffray, Sebastien, 38000, GRENOBLE (FR); Felix-Faure, Catherine, 38000, GRENOBLE (FR)
(74) Representative: Maureau, Philippe

(57) **Abstract**

The present invention relates to a container (1) intended to carry a product to be injected, comprising a globally tubular barrel (2) having an open proximal end and a substantially closed distal end provided with an outlet (3) for the passage of the product, said barrel comprising a restricted portion (5), a larger portion (6) distally spaced from said restricted portion, the inner diameter of said restricted portion being strictly less than the inner diameter of said larger portion, the barrel further comprising a proximal portion (7), proximally spaced from the restricted portion, the inner diameter of said proximal portion being strictly greater than that of the restricted portion.

## Description

The present invention relates to a container, prefilled or not, intended to carry a product to be injected and capable of receiving a stopper for sealing its proximal end, said container being intended to be used in connection with an injection device, said container allowing a smoother injection and improved storage conditions before injection.

In this application, the distal end of a component or of a device means the end furthest away from the hand of the user and the proximal end means the end closest to the hand of the user, when the component or device is in the use position. Similarly, in this application, the terms "in the distal direction" and "distally" mean in the direction of the injection, and the terms "in the proximal direction" and "proximally" mean in the direction opposite to the direction of injection.

Injection devices, such as syringes, are well known. Many different types of injection devices have been designed for administering medicines. Injection devices usually comprise a container intended to receive the product to be injected and a piston rod intended to move a piston within the container so as to expel the product therefrom at the time of injection. Pre-filled disposable injection devices are now widely used because they are very convenient and efficient and may be used directly in emergency cases.

Containers of prefilled injection devices usually contain medicines in a liquid state. In such cases, the container must be securely closed at both ends in order to safely store the medicine until use. The distal end of the container is usually provided with a sealing cap that may be removed just before injection. The proximal end of the container is usually provided with a stopper lodged within said container and forming a closure for said container.

Anyway, despite the presence of such stoppers at the proximal end of the containers, it may happen that leaks of products occur in known injection devices, as well as contamination from outside environment. Alternately, it is difficult to proceed to a smooth and regular injection with some prefilled containers because the force necessary to move the stopper is too high.

There is therefore a need for a container that would ensure a tight and secured sealing at its proximal end when prefilled with a medicine product, and this during all the time the product is stored before use, and at the same time that would allow a smooth injection.

An aspect of the present invention is to provide a container having a specific shape, so that closure of the container at its proximal end with a stopper may be ensured in an optimal way while authorizing to perform an optimal injection step in a smooth way.

Another aspect of the invention is to provide a prefilled container having a specific shape, closed at its proximal end with a stopper so that the closure of the container at its proximal end is ensured in an optimal way while authorizing to perform an optimal injection step in a smooth way.

The present invention relates to a container intended to carry a product to be injected, comprising a globally tubular barrel having an open proximal end and a substantially closed distal end provided with an outlet for the passage of the product, said barrel comprising at least a restricted portion, at least a larger portion distally spaced from said restricted portion, the inner diameter D1 of said restricted portion being strictly less than the inner diameter D2 of said larger portion, the barrel further comprising a proximal portion, proximally spaced from the restricted portion, the inner diameter D3 of said proximal portion being strictly greater than D1.

The invention further relates to a container filled with a product to be injected, comprising a globally tubular barrel having an open proximal end and a substantially closed distal end provided with an outlet for the passage of the product, said outlet being closed by a cap, said barrel comprising at least a restricted portion and at least a larger portion distally spaced from said restricted portion, the inner diameter D1 of said restricted portion being strictly less than the inner diameter D2 of said larger portion, the proximal end of said barrel being closed by means of a stopper being at least partially received within said restricted portion, at least a part of said stopper being in fluid-tight engagement with the wall of said restricted portion.

By "globally tubular barrel", it is meant, according to the present application that the barrel has an elongated shape having the general shape of a tube; such a tube may not have the same diameter along its entire length and may comprise tapered portions for example, such a tube may comprise regular linear tubular portions, non linear tubular portions, regular or non regular tapered portions, as long as the barrel is hollow and elongated.

The larger portion of the container of the invention will usually receive all or at least part of the product to be stored and then injected : as such the larger portion is preferably of substantially tubular shape, although it could alternately be slightly tapered and although its wall may not be totally linear along the entire length of the larger portion : in this view, in the present application, the larger portion defining a larger interior volume, the inner diameter D2 corresponds to the diameter of the largest cylinder capable of being inscribed in said larger interior volume. The product to be stored in the container of the invention is usually a liquid product.

The restricted portion of the container of the invention is located proximally to the larger portion and is in particular intended to receive a stopper in fluid-tight engagement in order to close the container in its storage position. When the container of the invention is prefilled with the product, in particular a liquid product, the restricted portion receives at least part of the stopper, the stopper being then in fluid-tight engagement with the wall of said restricted portion. The restricted portion is therefore also preferably of substantially tubular shape : like for the larger portion, the wall of the restricted portion may be linear or not along the length of the restricted portion : in this view, in the present application, the restricted portion defining a restricted interior volume, the inner diameter D1 corresponds to the diameter of the largest cylinder capable of being inscribed in said restricted interior volume.

In "fluid-tight engagement of at least a part of the stopper with the wall of the barrel, for example with the wall of the restricted portion" means according to the present application that at least part of the stopper contacts the wall of the barrel in a sealing manner, so that the leaks of the product, in particular of the liquid product, are avoided at the location of this sealing contact, thereby preventing the product to escape from the container. In embodiments, the stopper is also in fluid-tight engagement with the wall of the larger portion of the barrel of the container, for example during an injection step.

When the proximal portion is present, its wall, like for the larger and restricted portions, may not be totally linear on the entire length of the proximal portion. As such, the proximal portion defining a proximal interior volume, the inner diameter D3 corresponds to the diameter of the largest geometric part capable of being inscribed in said proximal interior volume. For example, the proximal portion may be of substantially tubular shape, the largest geometric part inscribed in the proximal interior volume being in such a case a cylinder. Alternatively, the proximal portion may be a tapered portion, in which case the largest geometric part inscribed in the proximal interior volume is a frustoconical part and the inner diameter D3 has a varying value on the length of the proximal portion. In other embodiments, the proximal portion may be a combination of a tubular portion and a tapered portion.

As will appear clearly from the description below, the container of the invention allows the optimal use of the properties of a stopper intended to close the proximal end of such a container, in particular a stopper having at least a cylindrical part capable of being compressed radially. Indeed, thanks to the presence of the restricted portion of the barrel, the stopper is forced to adopt a restricted state when lodged within the barrel of the container of the invention, at the location of said restricted portion, thereby providing an efficient and tight sealing of the container. This tight sealing protects the product present in the container from the environment and provides for a secure closure of the container, preventing the product from leaking out of the container and inner contamination from outside environment.

In order to proceed to the injection, the stopper is moved in the distal direction towards and within the larger portion of said barrel, where, thanks to its capability of deformation, it may adopt a less restricted state, allowing a smooth displacement of said stopper along the length of said larger portion: yet, in this use position of the container, the stopper may still provide the necessary tight contact with the inner surface of the barrel wall, thereby ensuring that the product present in the container is prevented from bypassing said stopper towards the proximal direction and is correctly ejected through the distal outlet and/or the needle. Indeed, during the injection step, the stopper may be in fluid-tight engagement with the wall of the larger portion.

When present, the proximal portion of the container of the invention allows introducing the stopper in the barrel in a more expanded state than that required for placing it within the restricted portion, thereby facilitating the step of closing the prefilled container for example. In embodiments, the larger portion and the proximal portion are tubular portions and D2 = D3. Such a barrel has the advantage of being easy to produce from a regular cylindrical tube, especially when the tube is made of glass, by simply forming a restricted portion in the middle region of the tube. In further embodiments, the proximal portion is a combination of a tubular portion and a tapered portion. For example, the tapered portion may be located between said restricted portion and said tubular portion, the proximal portion having then a varying diameter D3, D3 being strictly greater than D1. Such embodiments allow a progressive and simplified introducing of the stopper first in the proximal portion and then in the restricted portion.

In embodiments, the ratio D1/D2 ranges from 0.5 to strictly less than 1, preferably from 0.8 to strictly less than 1, and more preferably from 0.95 to strictly less than 1. Such values for the ratio D1/D2 give good results in term of gliding. The gliding is the force created by the friction of the stopper on the inner surface of the wall of the barrel forming the container. Indeed, the initiation of the gliding when the stopper is in the restricted portion requires usual gliding forces superior to the gliding forces along the larger portion. Having such a difference in the gliding forces along the larger and the restricted portions, as provided by the values of said ratio D1/D2, insures both safe sealing of the container and smooth injection, with a fluid-tight engagement of the stopper in the restricted portion of the barrel, as well as preferably in the larger portion of the barrel.

In embodiments, the barrel comprises a tapered portion located between the larger portion and the restricted portion, the inner diameter of the tapered portion ranging from D1 to D2. Like for the other portions defined above, said tapered portion defining a tapered interior volume, the inner diameter of the tapered portion corresponds to the diameter of the frustoconical part inscribed in said tapered interior volume. Such an embodiment provides for a progressive expansion of the stopper when acted upon, from the restricted portion, in which the stopper is in a restricted state in order to ensure a tight sealing, to the larger portion, where the stopper is in a less restricted state, such as an intermediate state. The progressive expansion of the stopper makes it simpler and smoother for the user to activate the stopper and to push distally thereon, via a piston rod for example. In consequence, the injection step is more controlled, the gliding of the stopper along the wall of this tapered portion is easier, and risks of jerkily injections are limited. On top of this benefit, such a tapered portion prevents proximal leakages when being used.

In embodiments, said barrel is made of one single piece. For example, the restricted portion, the larger portion, as well as the proximal portion and the tapered portion when these portions are present, are all part of a single piece, made of glass or of molded plastic.

In embodiments, the dimensions of the barrel are such that the ratio (d' + d")/L is greater than or equal to 0.1, preferably greater than or equal to 0.5, and more preferably greater than or equal to 0.75, in which d' designates the length of the larger portion, d" designates the length of the proximal portion, and L designates the total length of the barrel. Such values ensure both an optimal tightness during the shelf life of the container and a smooth movement of the stopper during injection, while preserving a significant volume for storage of the product, such as a liquid medicine, to be injected.

The container of the invention may comprise a stopper, intended to be or being at least partially received within said restricted portion so that at least a part of said stopper is in fluid-tight engagement with the wall of said restricted portion in a storage position of said container. In embodiments, the barrel being prefilled with a product, in particular a liquid product, and said outlet being closed by a cap, at least said part of said stopper is located within said barrel facing the restricted portion, thereby closing the proximal end of said container in a storage position of said container. The stopper may also be in fluid-tight engagement with the wall of said larger portion of said container during an injection step. The stopper may have a globally cylindrical shape of length Ls, and at least said part of said stopper may be capable of deforming from an expanded state, in which no stress is applied on said part of said stopper and in which said part of said stopper has an average outer diameter Dse, to a restricted state, in which radial compression forces are applied to said part of said stopper, and said part of said stopper has an average outer diameter Dsr equal to D1, Dse being strictly greater than D1 and Dse being greater than or equal to D2. Between its restricted state and its expanded state, said part of said stopper may adopt an intermediate state in which its outer average diameter is designed by Dsi, Dsi varying from Dsr to Dse.

Such part of said stopper may be made of elastomeric material such as rubber or thermoplastics.

In the container of the invention, the presence of a restricted portion, a larger portion and optionally a proximal portion as defined above, in combination with a stopper as defined above, allows to obtain different gliding forces depending on the location of the stopper within the barrel. In particular, for example, the ratio D1/D2 of the container of the invention, being less than 1 but equal to or greater than 0.5, preferably equal to or greater than 0.8 and again preferably equal to or greater than 0.95, in combination with the stopper as defined above, allows different gliding forces in the restricted portion and in the larger portion, so that the prefilled container of the invention is safely closed in its storage position, so as to both protect the product present in the container from the environment and prevent undesired leakages of the product, and also permits a smooth and regular injection process, the stopper being preferably in fluid-tight engagement with the wall of the larger portion during injection.

In embodiments, said stopper comprises one or more lips distributed on its length Ls, at least one of said lips, preferably at least two of said lips, forming said part of said stopper. In other words, at least one lip, preferably at least two lips, of the stopper are in fluid-tight engagement with the wall of the restricted portion. In embodiments, the stopper comprises three lips distributed on its length Ls, and at least two lips, preferably the three lips, form said part of the stopper and are therefore in fluid-tight engagement with the wall of the restricted portion.

In embodiments, said container having a longitudinal axis A, said restricted portion has a length greater or equal to the length of said stopper, said lengths being measured along said longitudinal axis. In such a case, the stopper may be totally received within the restricted portion in the storage position of the container. For example, the stopper is in fluid-tight engagement with the wall of the restricted portion on its entire length Ls in the storage position of the container. This provides good stability of the stopper in the storage position of the container : movements of the stopper are minimized despite external forces or differences in outside pressure that may arise from storage and transportation of the container. Moreover, this also ensures the good positioning of the stopper during manufacturing, especially after the container is filled with a pharmaceutical solution.

Alternately, the restricted portion may have a length less than or equal to that of the stopper.

In embodiments, the ratio Dsr/Dse ranges from 0.25 to 0.95, preferably from 0.5 to 0.95. Such values for the ratio Dsr/Dse provide for a stopper capable of optimally deforming from the restricted portion to the larger portion so as to ensure both safe sealing of the container in its storage position and smooth movement of the stopper during the injection step. In particular, such a ratio, depending on the nature of the stopper and in particular in combination with the ratio D1/D2 of the container of the invention, being less than 1 but equal to or greater than 0.5, preferably equal to or greater than 0.8 and again preferably equal to or greater than 0.95, provides for a good and adequate range for the average outer diameter of the stopper, allowing said stopper to perform efficiently its function of closure in its restricted state and to be in fluid-tight engagement with the wall of the restricted portion of the container of the invention, and to glide smoothly and sealingly along the inner wall of the larger portion during the injection step.

In embodiments, the inner surface of the barrel is coated with a lubricant to enhance the gliding of the stopper. Any convenient lubricant may be used. For example, the lubricant is selected from silicone, fluoropolymers such as polytetrafluoroethylene (PTFE) and combinations thereof. Such an embodiment provides for a smooth gliding of the stopper along the barrel wall during the injection step. In embodiments, the inner surface of the barrel is coated with silicone.

Another aspect of the present invention relates to an injection device comprising a container as described above, said container being prefilled or not.

The present invention will now be described in greater detail with the aid of the following description and the appended drawings in which:
- Figure 1 is a section view of a first embodiment of the container of the invention,
- Figure 2 is a section view of a second embodiment of the container of the invention,
- Figure 3 is a section view of an embodiment of the prefilled container of the invention,
- Figures 4A to 4C are section views of a stopper suitable for the container of the invention, respectively in its expanded state, an intermediate state and its restricted state,
- Figures 5A and 5B are partial section views of a third embodiment of the container of the invention,
- Figures 6A and 6B are partial section views of a fourth embodiment of the container of the invention,
- Figures 7 and 8 are section views of an embodiment of the container of the invention provided with a stopper and prefilled, respectively in a storage position and in the use position,
- Figures 9-11 are section views of the container of Figure 1, provided with a stopper and prefilled, used in connection with an injection device, respectively in a storage position and in two different positions of the injection step.

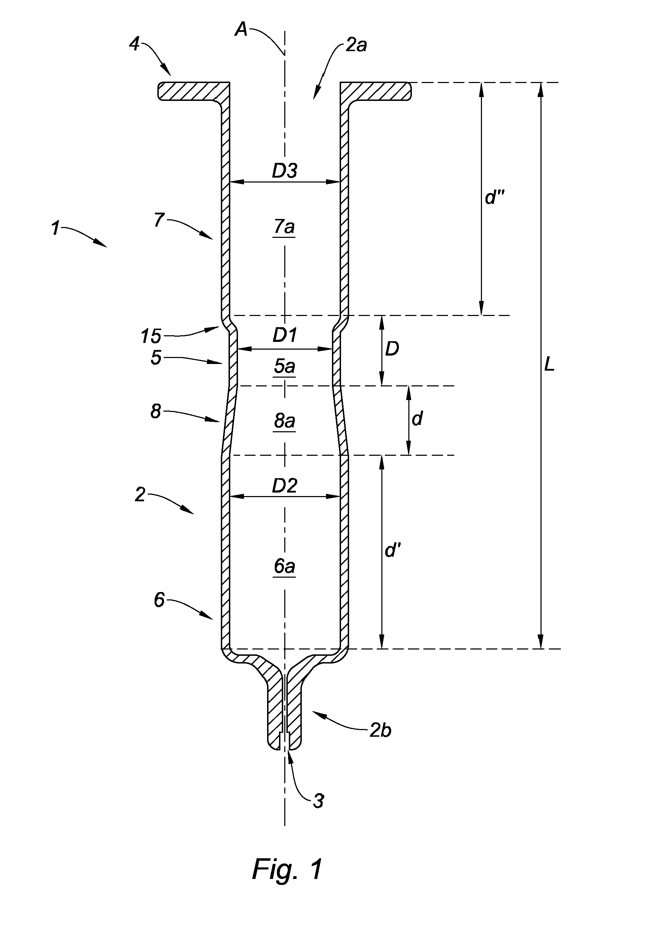

With reference to Figure 1 is shown a container 1 of the invention comprising a globally tubular barrel 2. The container 1 and the barrel 2 are aligned on a longitudinal axis A. The barrel 2 has an open proximal end 2a and a substantially closed distal end 2b provided with an outlet 3. As will appear in the description below, the barrel 2 is intended to carry a product, in particular a liquid product, for example a medicine or a pharmaceutical solution, to be injected, said product being intended to be expelled from the barrel 2 through the outlet 3.

In embodiments not shown, a needle is stacked on the outlet 3 and a cap closes the open end of the outlet 3 or of the needle.

The container 1 is intended to be in a storage position in which it is prefilled with the product and closed at its both ends, or in a use position, also called injection position, in which the product is being expelled through the outlet 3, thereby realizing the injection.

On the embodiment shown on Figure 1, the barrel 2 is further provided at its proximal end 2a with an outer flange 4 intended to form a bearing surface for the fingers of the user during the injection step. In embodiments not shown, the barrel has no outer flange and may be used as a cartridge for example, intended to be received within the body of an injection device.

The barrel 2 may be made out of any material suitable for the manufacture of containers intended to be filled with medicines, like plastic and/or glass.

With reference to Figure 1, the barrel 2 comprises a restricted portion 5 and a larger portion 6, distally spaced with respect to the restricted portion 5, both portions having substantially linear tubular shapes on the example shown : consequently the restricted portion 5 and the larger portion 6 define respectively a restricted interior volume 5a and a larger interior volume 6a, both having the shape of cylinders in the example shown. The inner diameter D1 of the restricted portion 5, which corresponds to that of the largest cylinder capable of being inscribed in the restricted interior volume 5a, is strictly less than the inner diameter D2 of the larger portion 6, which corresponds to that of the largest cylinder inscribed in the larger interior volume 6a.

The larger portion 6 is intended to receive at least part of the product present in the container. The length of the larger portion 6, measured along longitudinal axis A, may be determined with respect to its diameter in function of the volume of the dose of product to be stored and/or injected. On the example shown on Figure 1 , the length of the larger portion 6 is represented by d'.

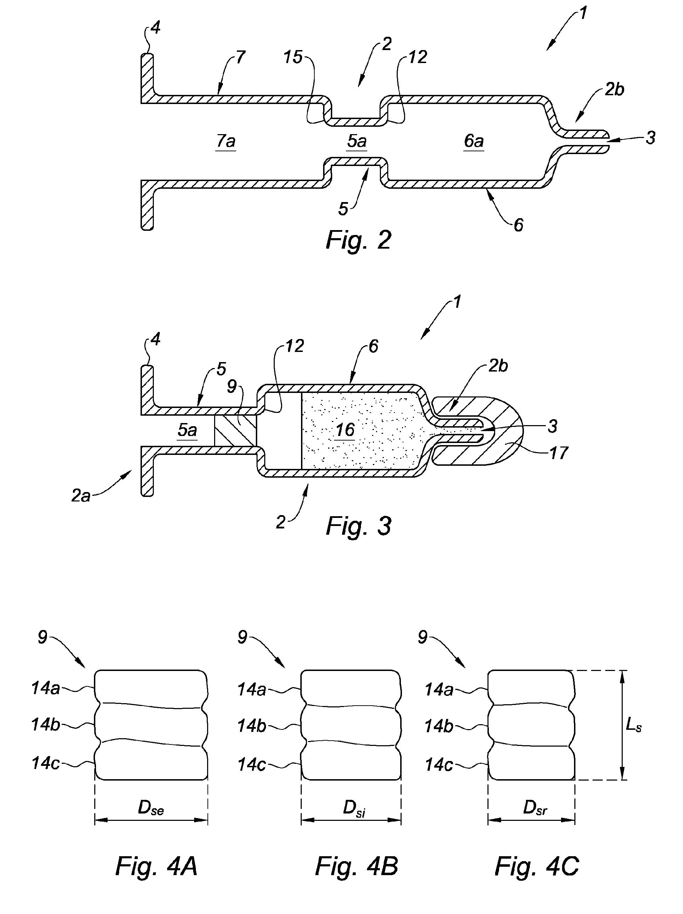

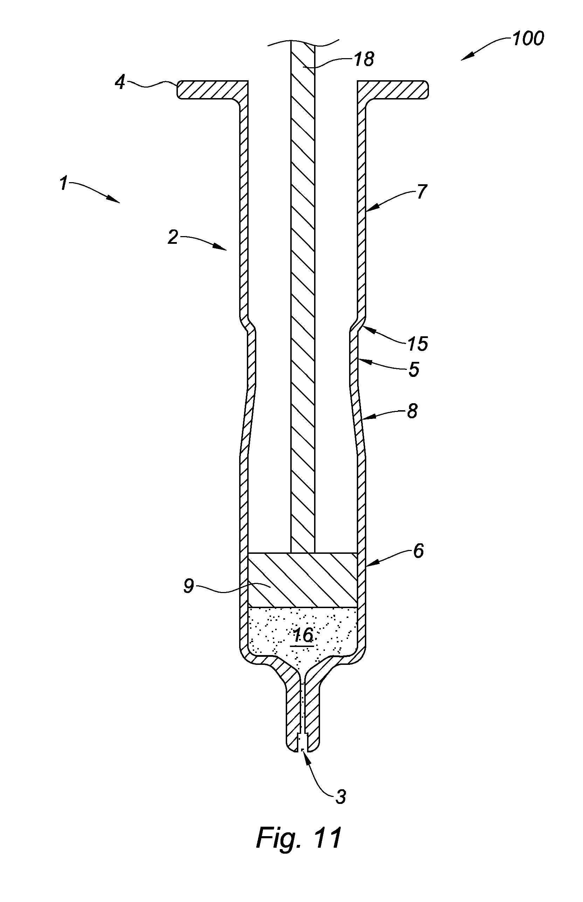

As will appear in the description below, the restricted portion 5 is intended to receive a stopper 9 (see Figures 4A-C and Figure 11) in the storage position of the container 1. The length of the restricted portion 5, measured along longitudinal axis A, may be greater than, less than or equal to the length of the stopper 9. On the example shown on Figure 1, the length of the restricted portion 5 is represented by D.

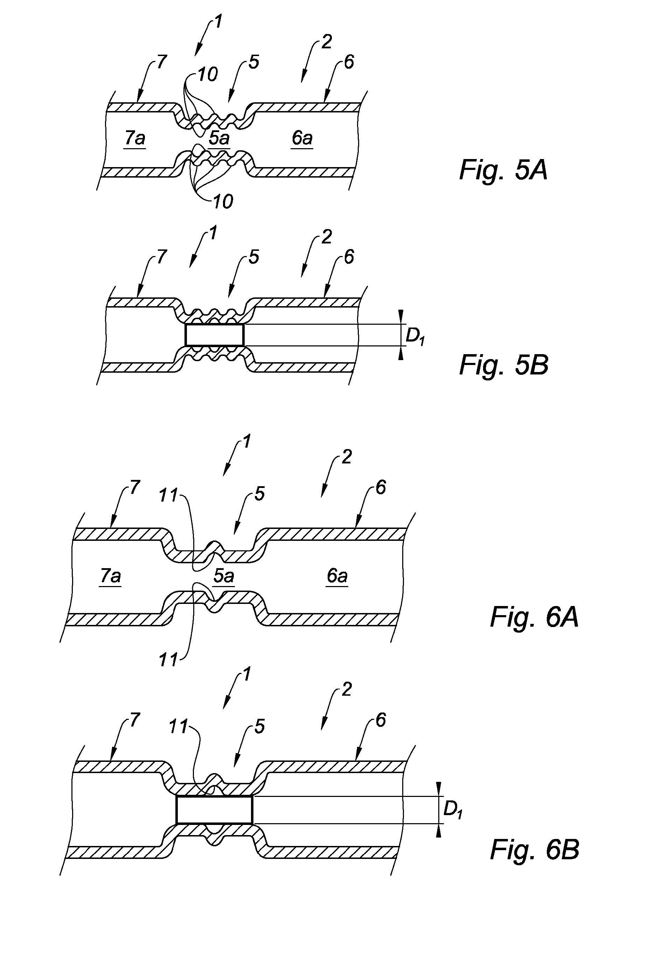

In alternative embodiments of the container 1 of the invention, the restricted portion 5 has a globally tubular shape but not linear : with reference to Figures 5A and 6A, on which the same references have been maintained for designating the same elements as in Figure 1, are shown such alternative embodiments. On Figure 5A is shown a restricted portion 5 having globally a tubular shape, the wall of which shows waves 10. As shown on Figure 5B, the waves 10 define a restricted interior volume 5a of the restricted portion 5, the largest cylinder capable of being inscribed in said restricted interior volume 5a having a diameter D1 : as explained above, according to the present application, in such an embodiment with a non linear shape of the wall of the restricted portion 5, D1 corresponds to the inner diameter of the restricted portion 5.

Alternately, on Figure 6A is shown a restricted portion 5 having globally a tubular shape, the inner wall of which shows an inner groove 11. As shown on Figure 6B, the inner wall of the restricted portion 5 together with the inner groove 11 define a restricted interior volume 5a of the restricted portion 5, the largest cylinder capable of being inscribed therein having a diameter D1. D1 is the inner diameter of the restricted portion 5.

In both alternative embodiments of Figures 5A and 6A, D2, which is not shown but which corresponds to the inner diameter of the larger portion 6 as shown on Figure 1, is greater than D1.

In embodiments with waves and grooves, as described above, the contact between a stopper, as shown on Figures 4A to 4C and as used as described in the present application, and the inner surface of the barrel 2 at the location of said restricted portion 5 is decreased and therefore, the initial force to initiate the stopper movement is reduced.

Alternately or in combination, the larger portion 6 as defined with reference to Figure 1 above also may have non linear tubular walls, for example wall showing waves or grooves or ridges.

With reference to Figure 1, the barrel 2 further comprises a proximal portion 7, proximally spaced from the restricted portion 5 : the proximal portion 7 also has a globally linear tubular shape and defines an interior proximal volume 7a having the shape of a cylinder on the example shown. The inner diameter D3 of the proximal portion 7, which corresponds to that of the largest cylinder capable of being inscribed in the proximal interior volume 7a is greater than D1. On Figure 1, the length of the proximal portion 7 is represented by d". The wall of the barrel 2 forms a shoulder 15 at the junction between the restricted portion 5 and the proximal portion 7.

In an embodiment not shown, the proximal portion 7 is a combination of a tubular portion and a tapered portion. For example, the tapered portion of the proximal portion replaces the shoulder 15 of Figure 1 and it links the tubular portion of the proximal portion to the restricted portion.

In an alternative embodiment shown on Figure 3, in which the same references designate the same elements as in Figure 1, the container 1 of the invention is prefilled with a product, in particular a liquid product 16, to be injected, and the barrel 2 has no proximal portion proximally to the restricted portion 5 : in such an embodiment, the outlet 3 is closed by a cap 17, the proximal end 2a of the barrel being closed by means of a stopper 9 such as the one of Figures 4A to 4C being received within the restricted portion 5, at least part of the stopper 9 being in fluid-tight engagement with the wall of the restricted portion 5. In the example shown, the length of the restricted portion 5 is greater than the length Ls (see Figure 4C) of the stopper 9. The stopper 9 is in fluid-tight engagement with the wall of the restricted portion 5 on its entire length Ls.

With reference to Figure 1, the barrel 2 further comprises a tapered portion 8 located between the restricted portion 5 and the larger portion 6. On the embodiment shown, the tapering of the tapered portion 8 is regular and this tapered portion 8 defines a tapered interior volume 8a, the inner diameter of the tapered portion 8, which corresponds to that of the frustoconical part inscribed in this tapered interior volume 8a, ranging from D1 to D2. On the example shown on Figure 1, the length of the tapered portion 8 is represented by d.

In an alternative embodiment of the container 1 of the invention shown on Figure 2, in which the same references designate the same elements as in Figure 1, the barrel 2 has no tapered portion located between the restricted portion 5 and the larger portion 6. In such embodiments, the wall of the barrel 2 may form a shoulder 12 at the junction between the restricted portion 5 and the larger portion 6.

Alternately or in combination, the proximal portion 7 and/or the tapered portion 8 as defined above may have non linear tubular walls, for examples wall showing waves or a groove or ridge, as shown on Figures 5A and 6A with reference to the restricted portion 5.

As appears on Figure 1, the total length of the barrel 2 of the container is : L = d' + d + D + d". By "total length" of the barrel 2, it is meant herein the length L of the tubular part of the barrel 2, said length L excluding the distal end 2b or tip of the barrel 2 as shown on Figure 1.

The ratio D1/D2 of the container 1 of the invention, whether it is prefilled or not, may range from 0.5 to strictly less than 1, preferably from 0.8 to strictly less than 1, and more preferably from 0.95 to strictly less than 1.

As will appear from the description below, this ratio allows obtaining two different gliding forces for a container of the invention provided with a stopper, on one hand between the restricted portion and the stopper in the storage position of the container, and on the other hand, between the larger portion and the stopper, in the use position of the container. The difference between these two gliding forces leads both to an optimal closure of the container in its storage position and to a smooth and regular injection process in the use position of the container, in other words during an injection step.

For example, the dimensions of the barrel are such that the ratio (d' + d")/L is greater than or equal to 0.1, preferably greater than or equal to 0.5, more preferably greater than or equal to 0.75. Such values ensure both an optimal tightness during the shelf life of the container and a smooth movement of the stopper during injection, while preserving a significant volume for storage of the product, such as a liquid medicine, to be injected.

For example, the containers of Figures 1-3 and 5A to 6B are intended to be used or are used with a stopper 9 as shown on Figures 4A to 4C, having a general cylindrical shape. As will be clear from the description below, the stopper 9 is intended to play the role of a proximal closure of the barrel 2 once said barrel 2 is prefilled with a product to be stored until injection. At the time of injection, the stopper 9 is then intended to be pushed distally in order to expel the product via the outlet 3, and preferably through a needle (not shown) for proceeding to the injection. The stopper 9 or portion of it is preferably made of a material allowing it to be compressed radially and inwardly so as to decrease its average outer diameter. For example, when no force is applied on the stopper 9 or portion of it, said stopper 9 or portion of it is in an expanded state, also corresponding to a rest state, in which it has an average outer diameter Dse, as shown on Figure 4A. The stopper 9 or portion of it is capable, under the effect of radial compression forces applied on its outer wall, to reach a restricted state, as shown on Figure 4C, in which it has an average outer diameter Dsr less than Dse. As will be clear in the description below, in the prefilled container of the invention, the stopper 9 or portion of it will be in its restricted state when it is received in the restricted portion 5, and therefore Dsr equals D1. Between these expanded state and restricted state, the stopper 9 or portion of it may be in an intermediate state, as shown on Figure 4B, in which it has an average outer diameter Dsi varying from Dse to Dsr. For example, the stopper 9 may be in an intermediate state when it is in the larger portion 6 of the container of the invention.

The stopper 9 according to the present invention may have a globally cylindrical shape: it may have one or more lips, thin or thick, with same or different diameters distributed on its length. The Figures 4A to 4C show a stopper 9 of globally cylindrical shape, having a length Ls and three thick lips, a proximal lip 14a, a central lip 14b and a distal lip 14d with similar outer diameters, said diameters corresponding to the average outer diameters Dse, Dsi and Dsr in the various states of the stopper 9, i.e. respectively in the expanded state, in the intermediate state and in the restricted state. The presence of three lips (14a, 14b, 14c) provides a good stability of the stopper 9 within the barrel 2.

The stopper 9 may be formed of elastomeric material such as rubber, thermoplastic, or material with expansion properties. Such stopper may consist of one or several materials, with or without coating : such coating may comprise silicone oil or fluoropolymer on one or several portions, or the totality of its surface.

Different kinds of stoppers may result in different gliding forces when these stoppers are received within the barrel 2 of a container 1 of the invention, depending on the expansion properties of said stoppers. Moreover, the ratio D1/D2 may be chosen according to these properties. For example, a stopper with a great expansion ability will allow a low D1/D2 ratio, i.e. close to 0.5. On the contrary, a rigid or semi-rigid stopper, with low expansion properties, may require a D1/D2 ratio greater than 0.95.

In embodiments, Dsr/Dse ranges from 0.25 to 0.95, preferably from 0.5 to 0.95. Such a ratio, depending on the nature of the stopper, provides for a good and adequate range for the average outer diameter of the stopper, allowing said stopper to perform efficiently its function of closure in its restricted state and to be in fluid-tight engagement with the wall of the restricted portion 5 of the container 1 of the invention, and to glide smoothly and sealingly along the inner wall of the larger portion 6 during the injection step.

As will appear from the description below, the radial compression forces required for moving the stopper 9, or at least the part of the stopper intended to be in fluid-tight engagement with the wall of the barrel 2, from its expanded state to one of its intermediate or restricted states may be provided by the inner wall of the tubular barrel 2.

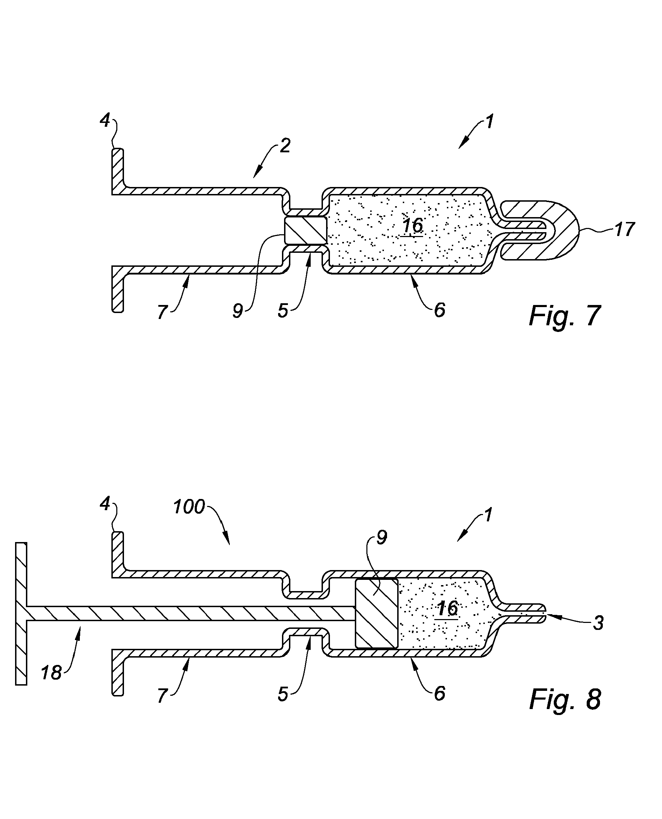

The implementation of the container 1 and stopper 9 of the invention, and their use as part of an injection device of the invention, will now be described in reference with Figures 7 and 8 in which the container 1 is similar to that of Figure 2 and the stopper 9 is similar to that of Figures 4A to 4C.

For the sake of clarity, the references D1, D2, D3, Dse, Dsr and Dsi are not reported on Figures 7 and 8 but they are as defined for Figures 1 and 4A to 4B described above. Moreover, given the regular shapes of the restricted portion 5 and larger portion 6 of the container of Figures 7 and 8, D1 corresponds to the inner diameter of restricted portion 5 and D2 corresponds to the inner diameter of larger portion 6. D3 corresponds to the inner diameter of the proximal portion 7, said proximal portion 7 having a tubular shape on the example shown.

With reference to Figure 7 is shown a container 1 similar to that of Figure 2, in a storage position : in such a position, the container 1 is prefilled with a product 16, such as a liquid medicine. The product 16 fills the interior volume of the larger portion 6 and the container 1 is closed at its distal end with a cap 17. Moreover, the stopper 9 is received within the restricted portion 5 thereby closing the larger portion 6 at its proximal end. In this location, the stopper 9 is in its restricted state, and the wall of the restricted portion 5 provides the radial compression forces for maintaining appropriate sealing with said stopper 9 in such restricted state with an average outer diameter of Dsr equal to D1.

Because of the restricted portion 5, the stopper 9 is in very tight contact with the inner wall of the barrel 2 at the location of the restricted portion 5, and in particular is in tight-fluid engagement with the wall of the restricted portion 5 on its entire length Ls (see Figure 4C). The stopper 9 therefore performs its closure function very efficiently and prevents the product 16 from leaking out of the larger portion 6, and therefore out of the container 1 in the storage position. Moreover, the stopper 9 further protects efficiently the product 16 from the outside environment.

Moreover, because of the presence of the proximal portion 7 having an inner diameter D3 greater than D1, the introduction of the stopper 9 in the proximal portion 7 and to the restricted portion 5 at the time of closing the container 1 is facilitated. Furthermore, in the case of a glass container, such a container may be obtained from a strictly linear glass cylinder, simply by restraining the diameter of said glass cylinder only on a portion of its total length.

When the user wishes to proceed with the injection of the product 16, he removes the cap 17 and provides the stopper 9 with a piston rod 18 for pushing distally said stopper 9, as shown on Figure 8, and, if required, connects a needle or a connector (not shown) to the outlet 3 thereby forming an injection device 100 according to the present invention.

Because Dse is greater than or equal to D2, the stopper 9 is in an intermediate state or expanded state when it enters the larger portion 6: in such a state, the stopper 9 is still in contact with the inner wall of the larger portion 6, although the gliding force here is less than the one required in the restricted portion 5 in the storage position of the container 1. This gliding force provides for secure contact with the inner wall of the larger portion 6 while allowing a smooth gliding of the stopper 9 along the wall of the larger portion 6.

The injection step may therefore be completed in a smooth way without any jerky steps.

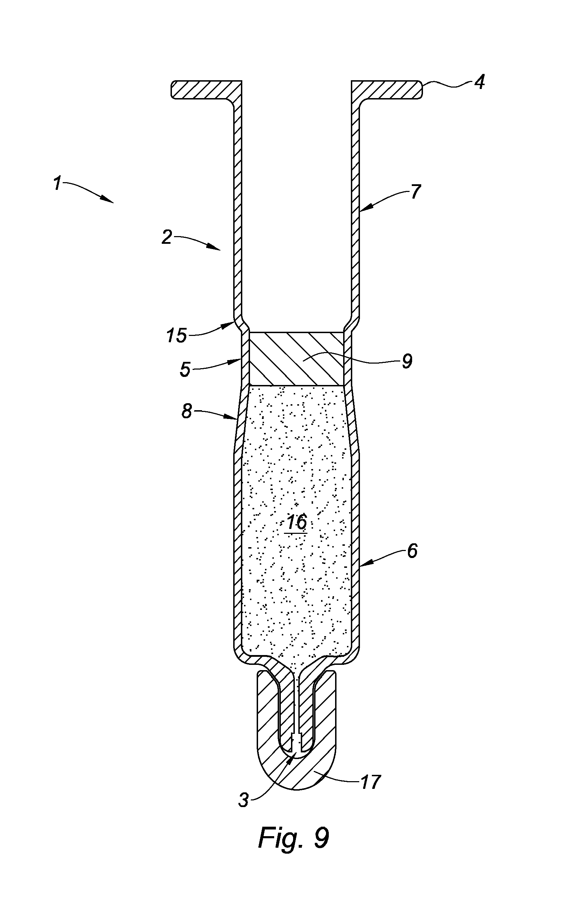

With reference to Figures 9-11, the container 1 of Figure 1 is used in combination with a stopper 9 as described in Figures 4A-4C.

With reference to Figure 9, the container 1 is in a storage position : in such a position, the container 1 is prefilled with a product 16, such as a liquid medicine. In this example, the product 16 fills the interior volume of the larger portion 6 and of the tapered portion 8, and the container 1 is closed at its distal end with a cap 17. Moreover, the stopper 9 is received within the restricted portion 5 thereby closing the proximal end of the volume of both the larger portion 6 and the tapered portion 8. In this location, the stopper 9 is in its restricted state, and the wall of the restricted portion 5 provides the radial compression forces for maintaining appropriate sealing with said stopper 9 in such restricted state with an average outer diameter of Dsr equal to D1.

Because of the relationship between D1 and Dse, the stopper 9 is in very tight contact with the inner wall of the restricted portion 5. The stopper 9 therefore performs its closure function very efficiently and prevents the product 16 from leaking out of the larger portion 6, and therefore out of the container 1 in the storage position.

Moreover, because of the presence of the proximal portion 7 having an inner diameter D3 greater than D1, the introduction of the stopper 9 first in the proximal portion 7, then to the restricted portion 5 at the time of closing the container 1 is facilitated.

In the example shown on Figure 9, the length D (see Figure 1) of the restricted portion 5 is greater than the length Ls of the stopper 9 and the stopper 9 is totally received within the restricted portion 5 and is in fluid-tight engagement with the wall of the restricted portion 5 on its entire length Ls (see Figure 4C). In particular, all three lips (14, 14b, 14c) of the stopper 9 (see Figures 4A-4C) are received within the restricted portion 5 and are in fluid-tight engagement with the wall of the restricted portion 5. In other embodiments not shown, the stopper 9 may be only partially received within the restricted portion 5 : for example, with reference to Figures 4A-4C, only the proximal lip 14a and the central lip 14b may be received within the restricted portion 5 and may be in fluid-tight engagement with the wall of the restricted portion 5, the distal lip 14c facing the tapered portion 8 and being therefore in an intermediate state or expanded state. In an alternative embodiment, only the distal lip 14c and the central lip 14b may be received within the restricted portion 5 and may be in fluid-tight engagement with the wall of the restricted portion 5, the proximal lip 14a facing the proximal portion 7, when present, and being therefore in an intermediate state or expanded state.

When the user wishes to proceed with the injection of the product 16, he removes the cap 17 and provides the stopper 9 with a piston rod 18 for pushing distally said stopper 9, as shown on Figure 12, and connects a needle or a connector (not shown) to the outlet 3, thereby forming an injection device 100 according to the present invention.

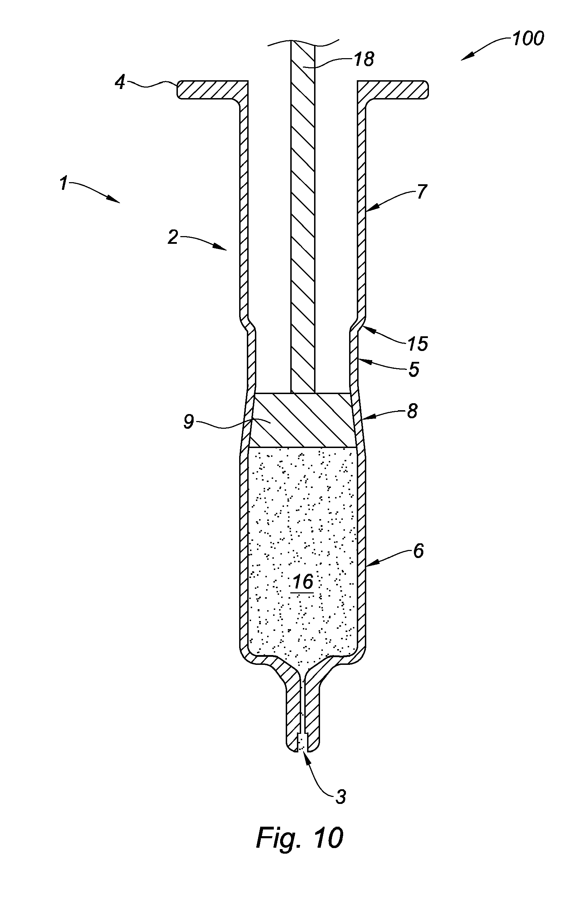

On Figure 10, the stopper 9 is still in the tapered portion 8 located between the restricted portion 5 and the larger portion 6. As is clear from the dimensions of the container 1, with the ratio D1/D2 strictly less than 1 but equal to or greater than 0.5, preferably equal to or greater than 0.8 and more preferably equal to or greater than 0.95, and (d'+d")/L greater than or equal to 0.1, preferably greater than or equal to 0.5 and more preferably greater than or equal to 0.75, the slope of the tapered portion 8 is low and the stopper 9 is allowed to progressively and slowly expand, going from its restricted state to an intermediate state in which its average outer diameter Dsi is greater than Dsr. As such, the stopper 9 maintains a good and secure contact with the inner wall of the tapered portion 8, thereby ensuring a safe and smooth injection. Indeed, the stopper 9 is preferably in fluid-tight engagement with the wall of the tapered portion 8. Moreover, the presence of the tapered portion 8 allows for a smooth starting of the stopper 9 right at the beginning of the injection : indeed, because the inner diameter of the tapered portion 8 at its junction with the restricted portion 5 is very close to that of said restricted portion 5, the stopper 9 is not allowed to expand quickly and dramatically when it is first acted upon to start the injection. Jerky injection steps are therefore avoided.

While the user continues to push distally on the piston rod 18, the stopper 9 reaches the larger portion 6, as shown on Figure 11, in which it expands a little more while still being maintained in an intermediate or expanded state in order to ensure an efficient sealing contact with the inner wall of the barrel 2 in the larger portion 6. Indeed, during the injection step, the stopper 9 is preferably in fluid-tight engagement with the wall of the larger portion 6.

Such an embodiment enables to perform a smooth and safe injection from the beginning to the end of the injection step.

In order to improve the smoothing of the injection step, the barrel 2 of the container 1 of the invention may be coated on its inner surface with a coating such as silicone, a fluoropolymer such as polytetrafluoroethylene (PTFE) or any other convenient lubricant. For example, the inner surface of the barrel 2 is coated with silicone. Such a coating improves the gliding of the stopper 9 along the inner wall of the barrel 2.

The shape of the container of the invention allows providing different gliding forces between the inner wall of the barrel and a stopper used both for closing said container when it is in its storage position and for expelling the product at the time of injection, depending on the location of the stopper within the various portions of the barrel. The container of the invention therefore allows providing a prefilled container particularly safe regarding the protection of the product stored with respect to the environment as well as with respect to the potential risks of leakage of the products. Moreover, the prefilled container of the invention provides for a smooth and regular injection step.

The examples below illustrate the invention.

### Example 1: 1 ml prefilled syringe

A container of the invention is provided with the following dimensions, with the definition of the parameters as shown on Figure 1:
- D1 = 6.05 mm
- D2 = D3 = 6.35 mm,
- L = 54 mm,
- D = 8 mm,
- d' = 30 mm,
- d"=12mm,
- d = 4 mm.

The volume of the pharmaceutical solution to be contained in the volume 6a of the larger portion 6 is at most 1 ml.

The ratio D1/D2 is therefore about 0.95.

The ratio (d' + d")/L is therefore about 0.78.

Such values ensure both an optimal tightness during the shelf life of the container and a smooth movement of the stopper as defined below during injection, while preserving a significant volume for storage of the product, such as a liquid medicine, to be injected.

Such a container is produced in glass starting from a strictly cylindrical hollow glass tube by a conventional process. An additional restriction step is added to obtain the restricted portion 5: the barrel is heated and set in a rotating movement against a fixed solid of the corresponding dimension.

After manufacturing the container itself, an injection device of the invention is obtained from said container : the container is filled in a sterile environment with a pharmaceutical solution and closed by a rubber stopper having the following dimensions :
- Dse = 6.40 mm,
- Dsr = D1 = 6.05 mm,

The ratio Dsr/Dse is therefore about 0.94.

Such a ratio provides for a good and adequate range for the average outer diameter of the rubber stopper of the present example, allowing said stopper to perform efficiently its function of closure of the container in its restricted state, and to be also in fluid-tight engagement with the wall of the restricted portion 5 of the container as well as to glide smoothly and sealingly along the inner wall of the larger portion 6 during the injection step. Indeed, during the injection step, the stopper is preferably in fluid-tight engagement with the wall of the larger portion 6.

Such a prefilled syringe presents a very good sealing and a high degree of pharmaceutical solution preservation, while maintaining a smooth gliding during the injection step.

### Example 2: 20 ml prefilled syringe

A container of the invention is provided with the following dimensions, with the definition of the parameters as shown on Figure 1:
- D1 = 18.05 mm
- D2 = D3 = 19.05 mm,
- L = 96.8 mm,
- D =20 mm,
- d'= 52 mm,
- d" = 19 mm,
- d = 5.8 mm.

The volume of pharmaceutical solution to be contained in the inner strictly cylindrical volume is at most 20 ml.

The ratio D1/D2 is therefore about 0.95.

The ratio (d' + d")/L is therefore about 0.73.

Such values ensure both an optimal tightness during the shelf life of the container and a smooth movement of the stopper as defined below during injection, while preserving a significant volume for storage of the product, such as a liquid medicine, to be injected.

Such a container is produced in plastic by injection molding with a widely known process. The restricted portion 5 is obtained by a specific shape of the molds.

After manufacturing the container itself, an injection device of the invention is obtained from said container : the container is filled in a sterile environment with a pharmaceutical solution and closed by a rubber stopper having the following dimensions :
- Dse = 19.25 mm,
- Dsr = D1 = 18.05 mm,

The ratio Dsr/Dse is therefore about 0.94.

Such a ratio provides for a good and adequate range for the average outer diameter of the rubber stopper of the present example, allowing said stopper to perform efficiently its function of closure of the container in its restricted state, and to be also in fluid-tight engagement with the wall of the restricted portion 5 of the container as well as to glide smoothly and sealingly along the inner wall of the larger portion 6 during the injection step. Indeed, during the injection step, the stopper is preferably in fluid-tight engagement with the wall of the larger portion 6.

Such a prefilled syringe presents a very good sealing and a high degree of pharmaceutical solution preservation, while maintaining a smooth gliding during the injection step.

## Claims

1. Container (1) intended to carry a product to be injected, comprising a globally tubular barrel (2) having an open proximal end (2a) and a substantially closed distal end (2b) provided with an outlet (3) for the passage of the product, said barrel comprising at least a restricted portion (5), at least a larger portion (6) distally spaced from said restricted portion, the inner diameter D1 of said restricted portion being strictly less than the inner diameter D2 of said larger portion, the barrel further comprising a proximal portion (7), proximally spaced from the restricted portion, the inner diameter D3 of said proximal portion being strictly greater than D1.

2. Container (1) filled with a product to be injected, comprising a globally tubular barrel (2) having an open proximal end (2a) and a substantially closed distal end (2b) provided with an outlet (3) for the passage of the product, said outlet being closed by a cap (17), said barrel comprising at least a restricted portion (5) and at least a larger portion (6) distally spaced from said restricted portion, the inner diameter D1 of said restricted portion being strictly less than the inner diameter D2 of said larger portion, the proximal end of said barrel being closed by means of a stopper (9) being at least partially received within said restricted portion, at least a part of said stopper being in fluid-tight engagement with the wall of said restricted portion.

3. Container (1) according to claim 1 or 2, wherein the ratio D1/D2 ranges from 0.5 to strictly less than 1, preferably from 0.8 to strictly less than 1, and more preferably from 0.95 to strictly less than 1.

4. Container (1) according to any one of claims 1 to 3, wherein the barrel comprises a tapered portion (8), located between the larger portion and the restricted portion, the inner diameter of said tapered portion ranging from D1 to D2.

5. Container (1) according to any one of claims 1 to 4, wherein the inner surface of the barrel is coated with a lubricant.

6. Container (1) according to claim 1, or according to any one of claim 3 to 5 when dependent on claim 1, further comprising a stopper (9) intended to be at least partially received within said restricted portion (5) so that at least a part of said stopper is in fluid-tight engagement with the wall of said restricted portion (5), in a storage position of said container.

7. Container (1) according to claim 6, wherein said barrel being prefilled with a product and said outlet being closed by a cap (17), at least said part of said stopper (9) is located within said barrel facing the restricted portion, thereby closing the proximal end of said container in a storage position of said container.

8. Container (1) according to any one of claims 2, 6 or 7, or according to any one of claims 3 to 5 when dependent on claim 2, wherein said stopper (9) is in fluid-tight engagement with the wall of said larger portion (6) of said container during an injection step.

9. Container (1) according to claim 8, wherein said stopper (9) having a globally cylindrical shape of length Ls, at least said part of said stopper is capable of deforming from an expanded state, in which no stress is applied on said part of said stopper and in which said part of said stopper has an average outer diameter Dse, to a restricted state, in which radial compression forces are applied to said part of said stopper and said part of said stopper has an average outer diameter Dsr equal to D1, Dse being strictly greater than D1 and Dse being greater than or equal to D2.

10. Container (1) according to claim 9, wherein the ratio Dsr/Dse ranges from 0.25 to 0.95, preferably from 0.5 to 0.95.

11. Container (1) according to claim or 10, wherein said stopper (9) comprises one or more lips (14a, 14b, 14c) distributed on its length Ls, at least one of said lips, preferably at least two of said lips, forming said part of said stopper.

12. Container (1) according to any one of claims 9 to 11, wherein said container having a longitudinal axis A, said restricted portion has a length greater or equal to the length Ls of said stopper (9), said lengths being measured along said longitudinal axis.

13. Container (1) according to claim 9 to 12, wherein in the storage position of the container, said stopper (9) is in fluid tight engagement with the wall of the restricted portion on its entire length Ls.

14. Container (1) according to claim 1, or according to any one of claims 3 to 13 when dependent on claim 1, wherein the dimensions of the barrel are such that the ratio (d' + d")/L is greater than or equal to 0.1, preferably greater than or equal to 0.5, and more preferably greater than or equal to 0.75, in which d' designates the length of the larger portion (6), d" designates the length of the proximal portion (7), and L designates the total length of the barrel (2).

15. Container (1) according to claim 1, or according to any one of claims 3 to 14 when dependent on claim 1, wherein said larger portion (6) and said proximal portion (7) being tubular portions, D2 = D3.

16. Injection device (100) comprising a container (1) according to any one of claims 1 to 15.
